# EUROPEAN PATENT APPLICATION

(11) **EP 4 039 316 A1**
(43) Date of publication of application: **10.08.2022**
(21) Application number: 20873283.4
(22) Date of filing: 16.09.2020
(51) Int. Cl.: A61M 25/09

(54) **GUIDE WIRE**

(30) Priority: 02.10.2019 JP 2019182149
(71) Applicant: ASAHI INTECC CO., LTD., Seto-shi, Aichi 489-0071 (JP)
(72) Inventor: UCHIMURA, Sho, Seto-shi, Aichi 489-0071 (JP); KUBOTA, Sho, Seto-shi, Aichi 489-0071 (JP)
(74) Representative: Prinz & Partner mbB
(86) International application number: PCT/JP2020/035038
(87) International publication number: WO 2021/065507

(57) **Abstract**

Provided is a guide wire including a core shaft, a coil body wound around the entire core shaft, a distal end joint portion that joins a distal end portion of the core shaft and a distal end portion of the coil body, a proximal end joint portion that joins a proximal end portion of the core shaft and a proximal end portion of the coil body, and a plurality of intermediate joint portions that are each arranged on a distal end side of a distal end of the proximal end joint portion and each join the core shaft and the coil body.

## Description

### TECHNICAL FIELD

The disclosed embodiments relate to a guide wire.

### BACKGROUND ART

There is known a guide wire used for inserting a medical device such as a catheter into a blood vessel, a digestive organ, or the like. Such a guide wire needs to have flexibility and restorability with respect to bending, torquability for transmitting an operation performed on the guide wire in a hand-grip portion to a distal end side, and a strong kink resistance with respect to deformation from breakage, distortion, and crushing. For example, Patent Literature 1 discloses a feature that the torquability of a guide wire is improved by tightly winding a torque transmission coil around an outer peripheral surface of a core wire (core shaft) on a proximal end side of the core wire.

### CITATION LIST

### Patent Literature

Patent Literature 1: US Patent Application Publication No. 2007/0049847

### SUMMARY OF THE INVENTION

### TECHNICAL PROBLEM

However, in the guide wire described in Patent Literature 1, there is a problem in that, when an operator grips the torque transmission coil and performs a rotating operation or a pushing operation, the torque at the hand-grip side cannot be properly transmitted to the distal end side, because the torque transmission force from the torque transmission coil to the core wire is weak. Further, in the rotating operation, the torque transmission coil may slip on the outer peripheral surface of the core wire, and thus, the torque transmission coil may be twisted, distorted, or crushed. It is noted that such a problem is not limited to an aspect in which an entire core shaft (core wire) is covered by a coil body (torque transmission coil), but is common to guide wires having an aspect in which a part of the core shaft on the proximal end side is not covered by the coil body and is exposed.

The disclosed embodiments have been contrived to solve at least some of the above-described problems, and an object of the disclosed embodiments is to improve torquability in a guide wire.

### SOLUTION TO PROBLEM

The disclosed embodiments have been contrived to solve at least some of the above-described problems, and can be implemented as the following aspects.
(1) According to one aspect of the disclosed embodiments, a guide wire is provided. The guide wire includes a core shaft, a coil body wound around the entire core shaft, a distal end joint portion that joins a distal end portion of the core shaft and a distal end portion of the coil body, a proximal end joint portion that joins a proximal end portion of the core shaft and a proximal end portion of the coil body, and a plurality of intermediate joint portions that are each arranged on a distal end side of a distal end of the proximal end joint portion and each join the core shaft and the coil body.
   According to the present configuration, the guide wire includes the plurality of intermediate joint portions that are each arranged on the distal end side of the proximal end joint portion, and join the core shaft and the coil body. Therefore, for example, even when an operator grips the coil body and performs a rotating operation or a pushing operation, a torque transmission force from the coil body to the core shaft can be improved by the plurality of intermediate joint portions, thus enabling transmission of an operation performed at a hand-grip side to the distal end side. As a result, according to the guide wire of the present configuration, it is possible to improve the torquability for transmitting the operation performed on the guide wire in the hand-grip portion to the distal end side. Further, even when the rotating operation is performed, the plurality of intermediate joint portions can prevent the coil body from slipping on an outer peripheral surface of the core shaft, and thus, it is possible to prevent the coil body from being twisted, distorted, or crushed.
(2) In the guide wire of the above-described aspect, between the proximal end joint portion and the intermediate joint portion arranged furthermost to the distal end side among the plurality of intermediate joint portions, remaining ones of the intermediate joint portions may be arranged at equal intervals.
   According to the present configuration, the intermediate joint portions are arranged at equal intervals, and thus, a force from the rotating operation or the pushing operation by the operator is easily transmitted to the distal end side, regardless of the position gripped by the operator. As a result, the torquability of the guide wire can be further improved.
(3) In the guide wire of the above-described aspect, the interval between one of the intermediate joint portions and an adjacent one of the intermediate joint portions may be 250 mm or less.
   According to the present configuration, the interval between one of the intermediate joint portions and an adjacent one of the intermediate joint portions is 250 mm or less, and thus, the force from the rotating operation or the pushing operation by the operator is more easily transmitted to the distal end side.
(4) In the guide wire of the above-described aspect, at least a part of the core shaft in a circumferential direction and at least a part of the coil body in the circumferential direction may be welded in the intermediate joint portion.
   According to the present configuration, the intermediate joint portion can be easily formed by welding at least a part of the core shaft in the circumferential direction and at least a part of the coil body in the circumferential direction.
(5) In the guide wire of the above-described aspect, the coil body includes a distal end side coil body arranged on the distal end side and a proximal end side coil body arranged on a proximal end side, and the intermediate joint portion arranged furthermost to the distal end side among the plurality of intermediate joint portions may join a proximal end portion of the distal end side coil body and a distal end portion of the proximal end side coil body.
   According to the present configuration, the guide wire includes the distal end side coil body and the proximal end side coil body, and thus, configurations (shapes, materials, etc.) of the distal end side coil body and the proximal end side coil body may be changed to obtain different characteristics at the distal end side and the proximal end side of the guide wire. Further, the distal end side coil body and the proximal end side coil body are joined by the intermediate joint portion, and thus, the distal end side coil body and the proximal end side coil body can be fixed. In addition, the distal end side coil body and the proximal end side coil body are joined by the intermediate joint portion arranged furthermost to the distal end side, and thus, the remaining intermediate joint portions can be arranged in the proximal end side coil body, a torque transmission force from the proximal end side coil body to the core shaft can be improved by the remaining intermediate joint portions, and the operation performed at the hand-grip side can be transmitted to the distal end side.
(6) In the guide wire of the above-described aspect, the proximal end side coil body may be a multi-thread coil in which a plurality of wires are wound into multiple threads.
   According to the present configuration, the proximal end side coil body is a multi-thread coil in which a plurality of wires are wound into multiple threads, and therefore, it is possible to further improve the torquability of the guide wire.
(7) In the guide wire of the above-described aspect, the proximal end side coil body may be a multi-thread coil in which a plurality of twisted wires obtained by twisting a plurality of wires are wound.
   According to the present configuration, the proximal end side coil body is a multi-thread coil in which a plurality of twisted wires obtained by twisting a plurality of wires are wound, and therefore, it is possible to further improve the torquability of the guide wire.
(8) In the guide wire of the above-described aspect, the distal end side coil body may be a single-thread coil in which one wire is wound into a single thread.
   According to the present configuration, the distal end side coil body is a single-thread coil in which one wire is wound into a single thread, and therefore, it is possible to improve the flexibility of the guide wire at the distal end side.

It is noted that the disclosed embodiments may be implemented in various aspects including, for example, a guide wire, a medical device including a guide wire, and a method for manufacturing the guide wire and the medical device including the guide wire.

### BRIEF DESCRIPTION OF DRAWINGS

Fig. 1 is an explanatory diagram illustrating a configuration of a guide wire according to a first embodiment.
Fig. 2 is an explanatory diagram illustrating a cross-sectional configuration taken along line A-A of Fig. 1.
Fig. 3 is an explanatory diagram illustrating a configuration of a guide wire according to a comparative example.
Fig. 4 is an explanatory diagram of a test method for a rotation followability test.
Fig. 5 is an explanatory graph of test results of the rotation followability test according to a comparative example.
Fig. 6 is an explanatory diagram of a gripping position in the rotation followability test.
Fig. 7 is an explanatory graph of test results of the rotation followability test.
Fig. 8 is an explanatory diagram illustrating a configuration of a guide wire according to a second embodiment.
Fig. 9 is an explanatory diagram illustrating a configuration of a guide wire according to a third embodiment.
Fig. 10 is an explanatory diagram illustrating a cross-sectional configuration taken along line B-B of Fig. 9.
Fig. 11 is an explanatory diagram illustrating a configuration of a guide wire according to a fourth embodiment.
Fig. 12 is an explanatory diagram illustrating a configuration of a guide wire according to a fifth embodiment.
Fig. 13 is an explanatory diagram illustrating a cross-sectional configuration taken along line C-C of Fig. 12.
Fig. 14 is an explanatory diagram illustrating a configuration of a guide wire according to a sixth embodiment.

### EMBODIMENTS OF THE INVENTION

### <First Embodiment>

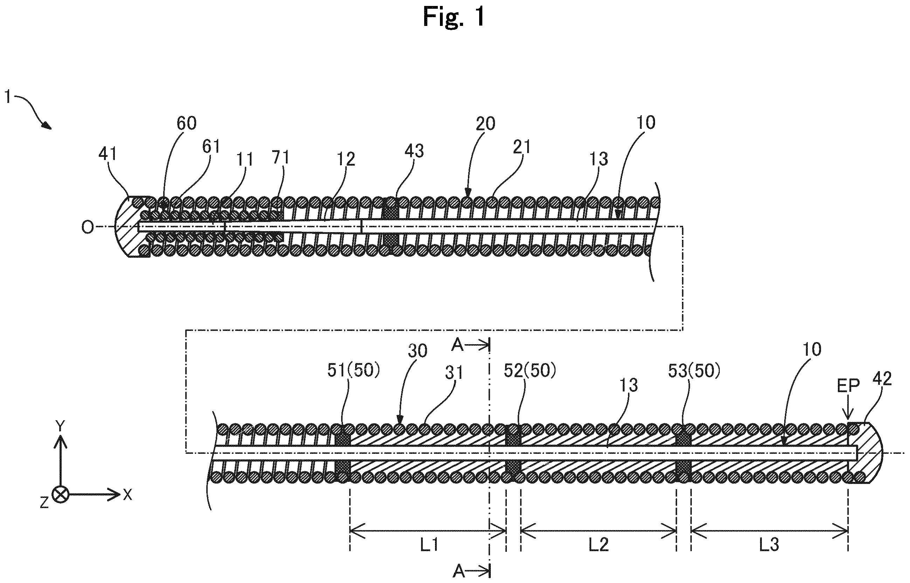

Fig. 1 is an explanatory diagram illustrating a configuration of a guide wire 1 according to a first embodiment. The guide wire 1 is a medical instrument used for inserting a device such as a catheter into a living body lumen, such as the vascular system, the lymphatic system, the biliary system, the urinary system, the respiratory system, the digestive system, secretory glands, and reproductive organs. The guide wire 1 includes a core shaft 10, a distal end side coil body 20, a proximal end side coil body 30, an inner coil body 60, a distal end joint portion 41, a proximal end joint portion 42, a first joint portion 43, a second joint portion 71, and a plurality of intermediate joint portions 50. By providing the guide wire 1 with the plurality of intermediate joint portions 50, it is possible to improve the torquability for transmitting an operation performed on the guide wire 1 in a hand-grip portion to a distal end side.

In Fig. 1, an axis passing through a center of the guide wire 1 is represented by an axial line O (dash-dot-dash line). In the example of Fig. 1, the axial line O coincides with each of axes passing through centers of the core shaft 10, the distal end side coil body 20, the proximal end side coil body 30, and the inner coil body 60. However, the axial line O may be different from each central axis of each of the above-described constituent components. Fig. 1 illustrates an X-axis, a Y-axis, and a Z-axis orthogonal to one another. The X-axis corresponds to a length direction of the guide wire 1, the Y-axis corresponds to a height direction of the guide wire 1, and the Z-axis corresponds to a width direction of the guide wire 1. The left side (- X-axis direction) in Fig. 1 is referred to as a "distal end side" of the guide wire 1 and each constituent component, and the right side in Fig. 1 (+ X-axis direction) is referred to as a "proximal end side" of the guide wire 1 and each constituent component. As for the guide wire 1 and each constituent component, an end located on the distal end side is referred to as a "distal end", and the distal end and the vicinity thereof are referred to as a "distal end portion". Further, an end located on the proximal end side is referred to as a "proximal end", and the proximal end and the vicinity thereof are referred to as a "proximal end portion". The distal end side corresponds to a "distal side" inserted into a living body, and the proximal end side corresponds to a "proximal side" operated by an operator such as a doctor. These denominations are common for the drawings from Fig. 1 onward.

The core shaft 10 is a tapered long member having a large diameter on the proximal end side and a small diameter on the distal end side. The core shaft 10 is arranged so as to extend coaxially with the axial line O. The core shaft 10 can be formed of a material such as a stainless alloy including SUS302, SUS304, SUS316, etc., a superelastic alloy including a nickel-titanium (NiTi) alloy, etc., a piano wire, a nickel-chromium alloy, a cobalt alloy, or tungsten. The core shaft 10 may be formed of a well-known material other than the above. The core shaft 10 includes a small diameter portion 11, a reduced diameter portion 12, and a large diameter portion 13, in this order from the distal end side to the proximal end side.

The small diameter portion 11 is provided on the distal end side of the core shaft 10. The small diameter portion 11 is a portion where an outer diameter of the core shaft 10 is smallest, and has a solid, substantially columnar shape having a constant outer diameter. The reduced diameter portion 12 is provided adjacent to the small diameter portion 11 on the proximal end side of the small diameter portion 11. The reduced diameter portion 12 has a substantially frustoconical shape having an outer diameter that decreases from the proximal end side toward the distal end side. The large diameter portion 13 is provided adjacent to the reduced diameter portion 12 on the proximal end side of the reduced diameter portion 12. The large diameter portion 13 has a larger diameter than the small diameter portion 11, and has a solid, substantially cylindrical shape. The outer diameter and the length of the small diameter portion 11, the reduced diameter portion 12, and the large diameter portion 13 can be freely determined. The shape of the small diameter portion 11, the reduced diameter portion 12, and the large diameter portion 13 can also be freely determined, and may be hollow or substantially polygonal columnar.

The distal end side coil body 20 is arranged on the distal end side of the guide wire 1. The distal end side coil body 20 has a substantially hollow cylindrical shape formed by spirally winding a wire 21 around the core shaft 10. Specifically, the distal end side coil body 20 is a single-thread coil in which one wire 21 is wound into a single thread. In the example of Fig. 1, the distal end side coil body 20 covers a part of the small diameter portion 11, the reduced diameter portion 12, and the large diameter portion 13 on the distal end side. The wire diameter of the wire 21 of the distal end side coil body 20, the average coil diameter of the distal end side coil body 20 (the average of the outer diameter and the inner diameter), and the length of the distal end side coil body 20 in a longitudinal direction can be freely determined. The wire 21 can be formed of, for example, a stainless alloy such as SUS304 and SUS316, a superelastic alloy such as a Ni-Ti alloy, etc., a piano wire, a radiolucent alloy such as a nickel-chromium alloy and a cobalt alloy, gold, platinum, tungsten, and a radiopaque alloy such as an alloy containing these elements (for example, a platinum-nickel alloy), or a well-known material other than the above.

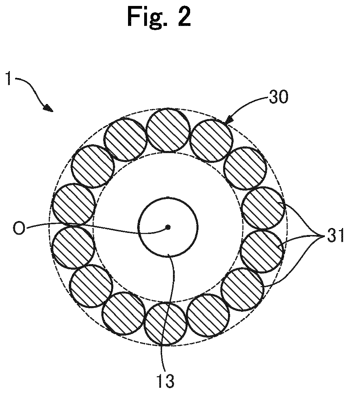

Fig. 2 is an explanatory diagram illustrating a cross-sectional configuration taken along line A-A of Fig. 1. As illustrated in Fig. 1, the proximal end side coil body 30 is arranged on the proximal end side of the guide wire 1. The proximal end side coil body 30 has a substantially hollow cylindrical shape formed by spirally winding a wire 31 around the core shaft 10. Specifically, the proximal end side coil body 30 is a multi-thread coil in which a plurality of the wires 31 are wound into multiple threads (Fig. 2). In the example of Fig. 2, the proximal end side coil body 30 formed by 14 of the wires 31 is illustrated, but the number of the wires 31 constituting the proximal end side coil body 30 can be freely determined. As illustrated in Fig. 1, the proximal end side coil body 30 covers a part of the large diameter portion 13 on the proximal end side, in other words, a remaining portion of the large diameter portion 13 that is not covered by the distal end side coil body 20. The wire diameter of the wire 31 of the proximal end side coil body 30, the average coil diameter of the proximal end side coil body 30, and the length of the proximal end side coil body 30 in the longitudinal direction can be freely determined. The wire 31 can be formed of a similar material as the wire 21. The material of the wire 31 may be the same as or different from that of the wire 21.

The distal end side coil body 20 and the proximal end side coil body 30 are collectively referred to as a "coil body". As illustrated in Fig. 1, in the guide wire 1 of the present embodiment, the coil body (the distal end side coil body 20 and the proximal end side coil body 30) is wound around the entire core shaft 10.

The inner coil body 60 has, on an inner side of the distal end side coil body 20, a substantially hollow cylindrical shape formed by spirally winding a wire 61 around the core shaft 10. The inner coil body 60 has a shorter length in the longitudinal direction (a direction of the axial line O) than the distal end side coil body 20, and is arranged on the distal end side of the distal end side coil body 20. In the example of Fig. 1, the inner coil body 60 covers the small diameter portion 11 and a part of the reduced diameter portion 12 on the distal end side. The wire diameter of the wire 61 of the inner coil body 60, the average coil diameter of the inner coil body 60, and the length of the inner coil body 60 in the longitudinal direction can be freely determined. The wire 61 can be formed of a similar material as the wire 21. The material of the wire 61 may be the same as or different from that of the wire 21.

It is noted that the inner coil body 60 may be a single-thread coil formed by winding one wire 61 into a single thread, may be a multi-thread coil formed by winding a plurality of the wires 61 into multiple threads, may be a single-thread twisted-wire coil formed by winding a twisted wire obtained by twisting a plurality of the wires 61 into a single thread, or may be a multi-thread coil (a multi-thread twisted-wire coil) formed by winding each twisted wire into multiple threads using a plurality of twisted wires obtained by twisting a plurality of the wires 61.

The distal end joint portion 41 is provided on the distal end portion of the guide wire 1, and integrally holds the core shaft 10 and the distal end side coil body 20 by joining the distal end portion of the core shaft 10 (specifically, the distal end portion of the small diameter portion 11) and the distal end portion of the distal end side coil body 20. The proximal end joint portion 42 is provided on the proximal end portion of the guide wire 1, and integrally holds the core shaft 10 and the proximal end side coil body 30 by joining the proximal end portion of the core shaft 10 (specifically, the proximal end portion of the large diameter portion 13) and the proximal end portion of the proximal end side coil body 30. The distal end joint portion 41 and the proximal end joint portion 42 can be formed of any bonding agent, for example, a metal solder such as silver solder, gold solder, zinc, a Sn-Ag alloy, an Au-Sn alloy, etc., or an adhesive such as an epoxy adhesive. The same bonding agent or different bonding agents may be used for the distal end joint portion 41 and the proximal end joint portion 42.

The first joint portion 43 is provided on a distal end side of the plurality of intermediate joint portions 50, and integrally holds the core shaft 10 and the distal end side coil body 20 by joining the core shaft 10 and the distal end side coil body 20. The second joint portion 71 is provided on a proximal end side of the inner coil body 60, and integrally holds the core shaft 10 and the inner coil body 60 by joining the core shaft 10 and the proximal end portion of the inner coil body 60. The first joint portion 43 and the second joint portion 71 can be formed of a similar material as the distal end joint portion 41. The material of the first joint portion 43 and the second joint portion 71 and the material of the distal end joint portion 41 may be the same or different.

The plurality of intermediate joint portions 50 are each arranged on a distal end side of a distal end EP of the proximal end joint portion 42, and each join the core shaft 10 and the proximal end side coil body 30. In the example of Fig. 1, the plurality of intermediate joint portions 50 include a first intermediate joint portion 51, a second intermediate joint portion 52, and a third intermediate joint portion 53.

The first intermediate joint portion 51 is arranged furthermost to the distal end side among the plurality of intermediate joint portions 50. The first intermediate joint portion 51 integrally holds the distal end side coil body 20, the proximal end side coil body 30, and the core shaft 10 by joining the proximal end portion of the distal end side coil body 20, the distal end portion of the proximal end side coil body 30, and the core shaft 10. The second intermediate joint portion 52 is arranged between the first intermediate joint portion 51 and the third intermediate joint portion 53 in the longitudinal direction (the direction of the axial line O). The third intermediate joint portion 53 is arranged furthermost to the proximal end side among the plurality of intermediate joint portions 50. The second intermediate joint portion 52 and the third intermediate joint portion 53 integrally hold the proximal end side coil body 30 and the core shaft 10 by joining the proximal end side coil body 30 and the core shaft 10. The plurality of intermediate joint portions 50 can be formed of a similar material as the distal end joint portion 41. The material of the plurality of intermediate joint portions 50 and the material of the distal end joint portion 41 may be the same or different.

As illustrated in Fig. 1, the remaining intermediate joint portions of the plurality of intermediate joint portions 50 (that is, the second intermediate joint portion 52 and the third intermediate joint portion 53) are arranged at equal intervals between the first intermediate joint portion 51 arranged furthermost to the distal end side and the proximal end joint portion 42. In other words, a length L1 between the proximal end of the first intermediate joint portion 51 and the distal end of the second intermediate joint portion 52, a length L2 between the proximal end of the second intermediate joint portion 52 and the distal end of the third intermediate joint portion 53, and a length L3 between the proximal end of the third intermediate joint portion 53 and the distal end EP of the proximal end joint portion 42 are all equal. Here, "equal" is not limited to the case where the lengths are completely the same, but includes an error to an extent at which a torque transmission performance described later can be achieved.

Hereinafter, the length L1 between the proximal end of the first intermediate joint portion 51 and the distal end of the second intermediate joint portion 52 will also be referred to as an interval L1 between the first intermediate joint portion 51 and the second intermediate joint portion 52. The length L2 between the proximal end of the second intermediate joint portion 52 and the distal end of the third intermediate joint portion 53 will also be referred to as an interval L2 between the second intermediate joint portion 52 and the third intermediate joint portion 53. The length L3 between the proximal end of the third intermediate joint portion 53 and the distal end EP of the proximal end joint portion 42 will also be referred to as an interval L3 between the third intermediate joint portion 53 and the proximal end joint portion 42. In the guide wire 1 of the present embodiment, the interval between one intermediate joint portion and an adjacent intermediate joint portion is 250 mm or less, that is, the intervals L1, L2, and L3, are all 250 mm or less.

### <Effect Example>

As described above, the guide wire 1 according to the first embodiment includes the plurality of intermediate joint portions 50 (the first intermediate joint portion 51, the second intermediate joint portion 52, and the third intermediate joint portion 53) that are each arranged on the distal end side of the proximal end joint portion 42 and join the core shaft 10 and the proximal end side coil body 30 (the coil body). Therefore, for example, even when the operator grips the proximal end side coil body 30 and performs a rotating operation or a pushing operation, a torque transmission force from the proximal end side coil body 30 to the core shaft 10 can be improved by the plurality of intermediate joint portions 50, thus enabling transmission of the operation performed at the hand-grip side to the distal end side. As a result, according to the guide wire 1 of the first embodiment, it is possible to improve the torquability for transmitting the operation performed on the guide wire 1 in the hand-grip portion to the distal end side. Further, even when the rotating operation is performed, the plurality of intermediate joint portions 50 can prevent the proximal end side coil body 30 from slipping on an outer peripheral surface of the core shaft 10, and thus, it is possible to prevent the proximal end side coil body 30 from being twisted, distorted, or crushed.

In the guide wire 1 of the first embodiment, the remaining second and third intermediate joint portions 52 and 53 of the plurality of intermediate joint portions 50 are arranged at equal intervals between the first intermediate joint portion 51 arranged furthermost to the distal end side and the proximal end joint portion 42 (Fig. 1: L1, L2, and L3). Therefore, the force from the rotating operation or the pushing operation by the operator is easily transmitted to the distal end side, regardless of the position gripped by the operator. As a result, the torquability of the guide wire 1 can be further improved. The interval between one intermediate joint portion and an adjacent intermediate joint portion is 250 mm or less, that is, the intervals L1, L2, and L3 are all 250 mm or less, and therefore, the force from the rotating operation or the pushing operation by the operator is more easily transmitted to the distal end side.

In addition, the guide wire 1 of the first embodiment includes the distal end side coil body 20 and the proximal end side coil body 30, and thus, the configurations (shapes, materials, etc.) of the distal end side coil body 20 and the proximal end side coil body 30 may be changed to obtain different characteristics at the distal end side and the proximal end side of the guide wire 1. The distal end side coil body 20 and the proximal end side coil body 30 are joined by the first intermediate joint portion 51 (the intermediate joint portion), and thus, the distal end side coil body 20 and the proximal end side coil body 30 can be fixed. In addition, the distal end side coil body 20 and the proximal end side coil body 30 are joined by the first intermediate joint portion 51 arranged furthermost to the distal end side, and thus, the remaining second and third intermediate joint portions 52 and 53 can be arranged in the proximal end side coil body 30, the torque transmission force from the proximal end side coil body 30 to the core shaft 10 can be improved by the remaining second and third intermediate joint portions 52 and 53, and the operation performed at the hand-grip side can be transmitted to the distal end side.

In the first embodiment, the proximal end side coil body 30 is a multi-thread coil in which a plurality of the wires 31 are wound into multiple threads (Fig. 2), and thus, it is possible to further improve the torquability of the guide wire 1. The distal end side coil body 20 is a single-thread coil in which one wire 21 is wound into a single thread, and therefore, it is possible to improve the flexibility of the guide wire 1 at the distal end side.

### <Rotation Followability Test>

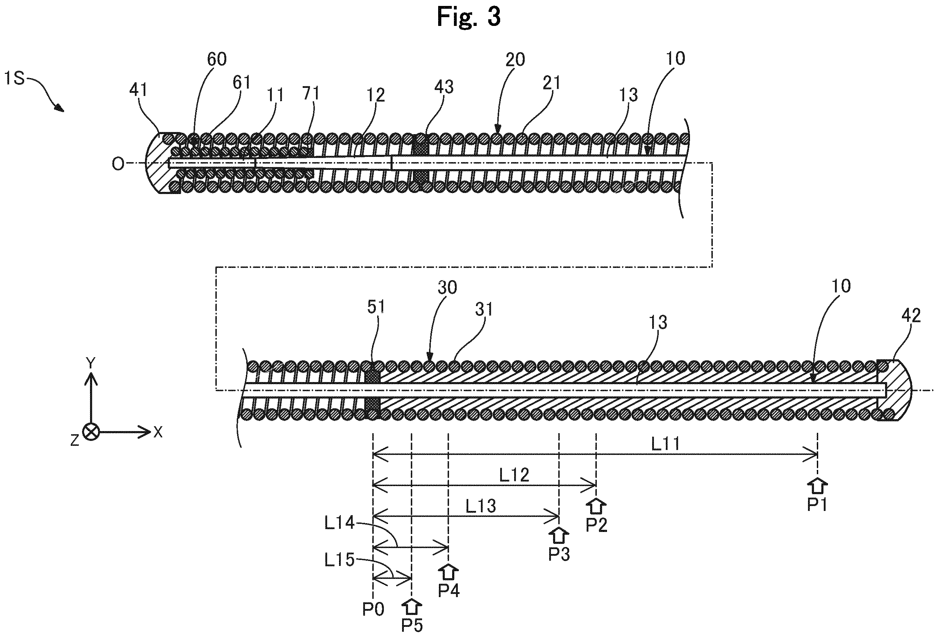

An improvement of the rotation following performance, that is, the torque transmission performance in the guide wire 1 of the first embodiment will be described with reference to Figs. 3 to 7. To prove an effect of the guide wire 1 of the first embodiment, a rotation followability test was carried out using a conventional guide wire 1S, as a comparative example. The rotation followability test is a test for quantitatively measuring the rotation following performance of the guide wires 1 and 1S.

Fig. 3 is an explanatory diagram illustrating a configuration of the guide wire 1S according to the comparative example. The guide wire 1S has the same configuration as the guide wire 1 described in Fig. 1, except that the guide wire 1S does not include a plurality of intermediate joint portions 50. The guide wire 1S includes only the first intermediate joint portion 51 that joins the distal end side coil body 20 and the proximal end side coil body 30, and does not include the second intermediate joint portion 52 and the third intermediate joint portion 53 described in Fig. 1. In other words, in the guide wire 1S, the distal end portion of the proximal end side coil body 30 is joined to the core shaft 10 by the first intermediate joint portion 51 and the proximal end portion of the proximal end side coil body 30 is joined to the core shaft 10 by the proximal end joint portion 42, but a portion between the distal end portion and the proximal end portion of the proximal end side coil body 30 is not joined to the core shaft 10.

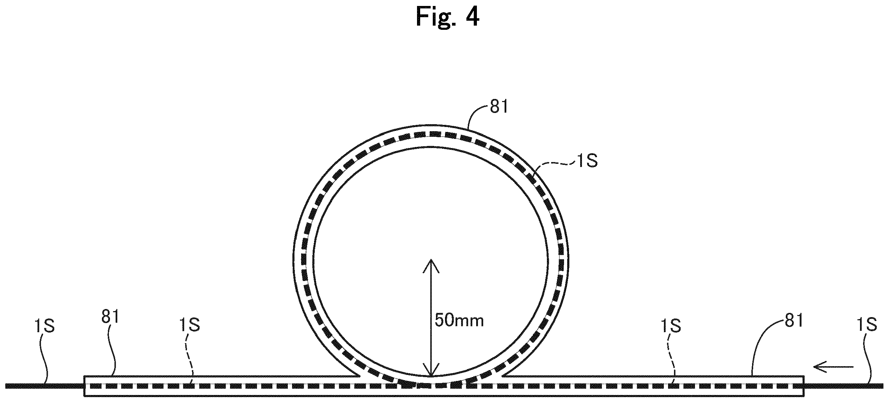

Fig. 4 is an explanatory diagram of a test method for the rotation followability test. In the rotation followability test, a circular ring having a radius of 50 mm was formed using a tube 81, and a test path was created in which straight portions were formed in front of and behind the ring. The guide wire 1S of the comparative example was inserted from one opening of the test path (an opening on the right side in Fig. 4). Subsequently, the guide wire 1S of the comparative example was pushed inward until the distal end protruded from the other opening of the tube 81. In this state, the guide wire 1S was rotated by gripping predetermined gripping positions (P1 to P5) on the proximal end side of the guide wire 1S, and the number of times the distal end side of the guide wire 1S rotated was measured.

The gripping positions P1 to P5 used in the rotation followability test will be described with reference to Fig. 3. The gripping position P1 was set to a position separated from a center P0 of the first intermediate joint portion 51 by a length L11 in the direction of the axial line O (Fig. 3: an open arrow P1). Similarly, the gripping position P2 was set to a position separated from the center P0 by a length L12, the gripping position P3 was set to a position separated from the center P0 by a length L13, the gripping position P4 was set to a position separated from the center P0 by a length L14, and the gripping position P5 was set to a position separated from the center P0 by a length L15 (Fig. 3: open arrows P2 to P5). Here, the lengths L11 to L15 can be freely determined as long as a relationship of L11 > L12 > L13 > L14 > L15 is satisfied. The gripping position P1 is closest to the proximal end joint portion 42, and the gripping position P5 is closest to the first intermediate joint portion 51.

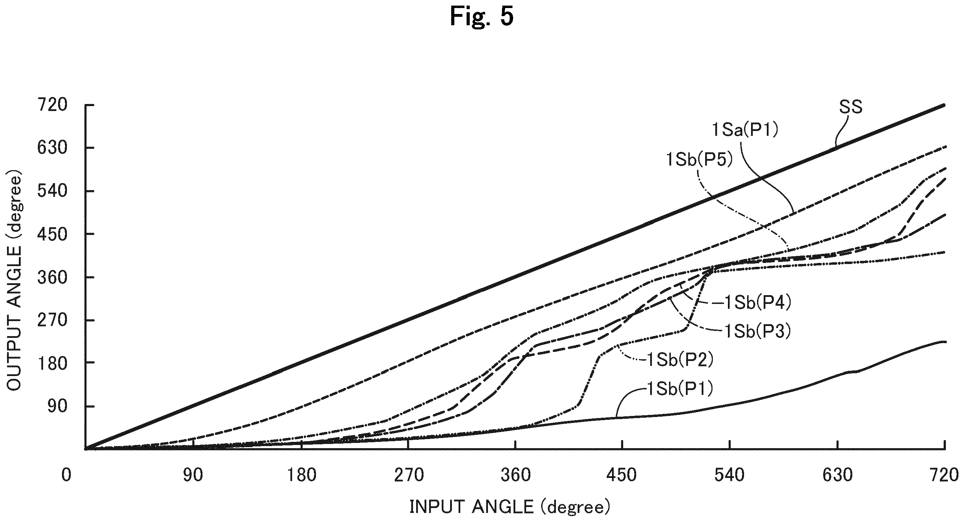

Fig. 5 is an explanatory graph of test results of the rotation followability test according to the comparative example. In Fig. 5, the horizontal axis indicates a rotation angle (an input angle: degree) on the proximal end side of the guide wire 1S of the comparative example, and the vertical axis indicates a rotation angle (an output angle: degree) on the distal end side of the guide wire 1S of the comparative example. Firstly, a plurality of the guide wires 1S (Fig. 3) of the comparative example were prepared, and the rotation followability test was conducted in a state where the gripping position P1 was gripped in each of the guide wires 1S. The results indicated that a guide wire 1Sa having a relatively excellent rotation following performance and a guide wire 1Sb having a relatively poor rotation following performance were obtained. In Fig. 5, the measurement result of the guide wire 1Sa is represented by a broken line having a narrow pitch (1Sa: P1), and the measurement result of the guide wire 1Sb is represented by a thin solid line (1Sb: P1). Further, in Fig. 5, an ideal value SS is represented by a thick solid line. The ideal value SS represents a state where the distal end side completely follows the rotation on the proximal end side.

Next, the rotation followability test was conducted by gripping each of the other gripping positions P2, P3, P4, and P5 described in Fig. 3 in the guide wire 1Sb having the relatively poor rotation following performance. In Fig. 5, a measurement result at the gripping position P2 is represented by a two-dot chain line (1Sb: P2), a measurement result at the gripping position P3 is represented by a dash-dot-dash line having a wide pitch (1Sb: P3), a measurement result at the gripping position P4 is represented by a broken line having a wide pitch (1Sb: P4), and a measurement result at the gripping position P5 is represented by a dash-dot-dash line having a narrow pitch (1Sb: P5).

In the guide wire 1Sb, at the gripping position P5 closest to the first intermediate joint portion 51, the output angle follows the input angle, so that the rotation following performance is relatively high. Further, at the gripping position P4 located next closest to the first intermediate joint portion 51 and the gripping position P3 located second next closest, although slightly delayed, the output angle follows the input angle, so that the rotation following performance is maintained. On the other hand, at the gripping position P4, the output angle is delayed with respect to the input angle. This indicates that the rotation at the proximal end side is not transmitted to the distal end side in real time, and when the torque is accumulated for a while and then suddenly released, the distal end side starts rotating (a state where a so-called "rebound" occurs). Thus, the rotation following performance is relatively low at the gripping position P4.

As described above, in the guide wire 1S of the comparative example, even when the gripping position P1 near the proximal end joint portion 42 is gripped, unfortunately, both the guide wire 1Sa having a relatively excellent rotation following performance and the guide wire 1Sb having a relatively poor rotation following performance are obtained. Further, in the guide wire 1Sb having the relatively poor rotation following performance, it can be seen that the rotation following performance may vary depending on which of the gripping positions P2 to P5 the operator grips, and the rotation following performance may decrease.

After the test of the guide wire 1S of the comparative example, the rotation followability test was also performed on the guide wire 1 of the first embodiment by using a method similar to that of the comparative example. Specifically, first, a plurality of the guide wires 1 described in Fig. 1 were prepared, and a rotation followability test was carried out for each by the method described in Fig. 4 while gripping the same position as the gripping position P1 of Fig. 3 in each of the guide wires 1 (a position separated from the center P0 of the first intermediate joint portion 51 by a length L11 in the direction of the axial line O). Subsequently, for the guide wire 1b having the relatively poor rotation following performance, a rotation followability test was further performed for each of gripping positions P6 and P7 described below.

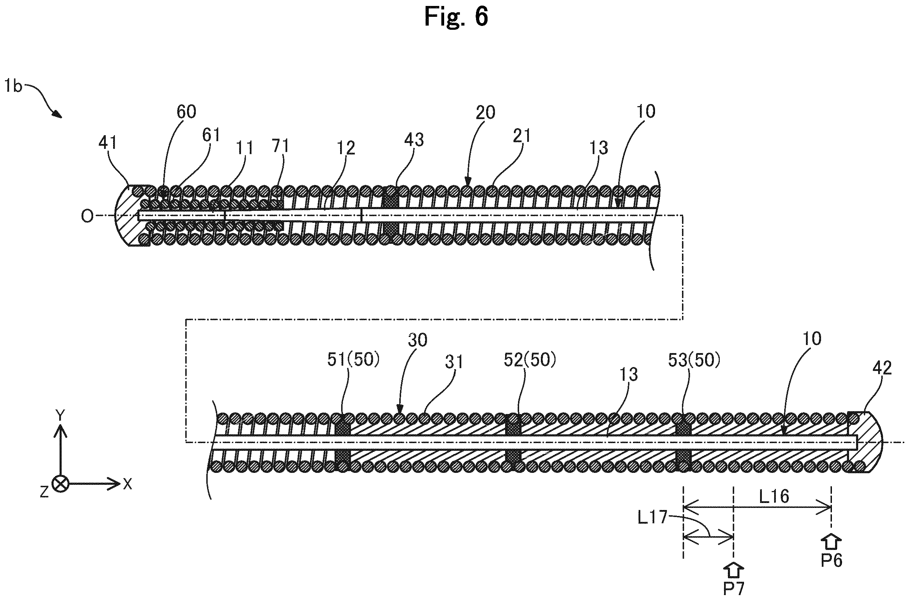

Fig. 6 is an explanatory diagram of the gripping positions P6 and P7 in the rotation followability test. The gripping position P6 was set to a position separated by a length L16 in the direction of the axial line O from the center P0 of the intermediate joint portion located furthermost to the proximal end side (the third intermediate joint portion 53 in the example illustrated in the drawing) among the plurality of intermediate joint portions 50 (Fig. 6: an open arrow P6). Further, the gripping position P7 was set to a position separated from the center P0 by a length L17 (Fig. 6: an open arrow P7). Here, the lengths L16 and L17 can be freely determined as long as a relationship of L16 > L17 is satisfied. The gripping position P6 is closest to the proximal end joint portion 42, and the gripping position P7 is closest to the third intermediate joint portion 53.

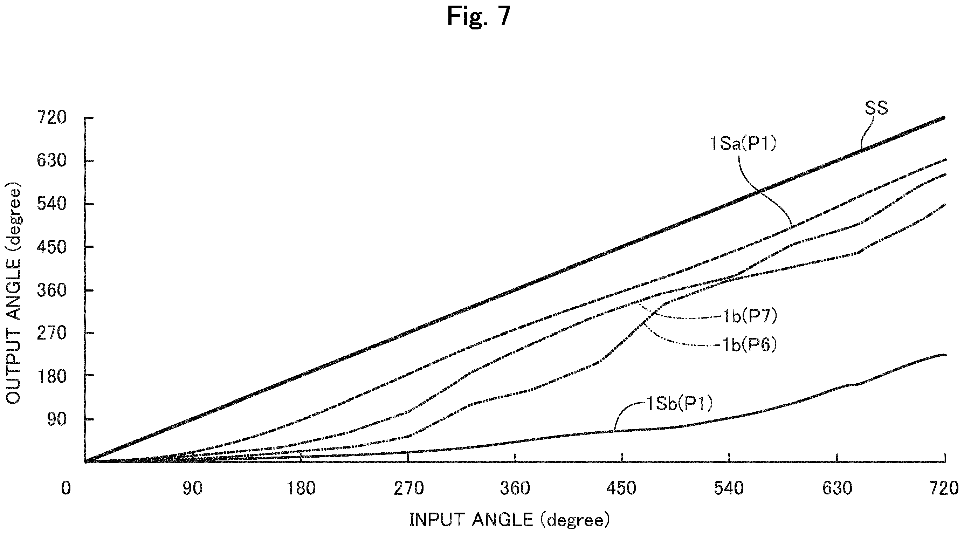

Fig. 7 is an explanatory graph of test results of a rotation followability test. In Fig. 7, the horizontal axis indicates a rotation angle (input angle: degree) on the proximal end side of the guide wire 1b described above (the guide wire 1b having the relatively poor rotation following performance among the guide wires 1 described in the first embodiment), and the vertical axis indicates a rotation angle (output angle: degree) on the distal end side of the guide wire 1b. In Fig. 7, a measurement result at the gripping position P6 of the guide wire 1b is represented by a two-dot chain line (1b: P6) and a measurement result at the gripping position P7 of the guide wire 1b is represented by a dash-dot-dash line (1b: P7). For convenience of explanation, Fig. 7 represents the test result of the guide wire 1Sa having the relatively excellent rotation following performance (1Sa: P1), the test results of the guide wire 1Sb having the relatively poor rotation following performance (1Sb: P1), and the ideal value SS described in Fig. 5.

In the guide wire 1b, at the gripping position P7 closest to the first intermediate joint portion 51, the output angle follows the input angle, so that the rotation following performance is relatively high. Further, at the gripping position P6 that is close to the proximal end joint portion 42 while being relatively far from the first intermediate joint portion 51, the output angle gently follows the input angle, so that the rotation following performance is relatively high.

From the rotation followability tests described above, it can be seen that, in the guide wire 1 described in the first embodiment, as described in Fig. 7, a high rotation following performance (torque transmission performance) can be maintained in both of the gripping positions P6 and P7, even in the example (guide wire 1b) in which the rotation following performance is relatively poor. This result is achieved because of the fact that the interval between the gripping positions P6 and P7 and the nearest joint portion (the proximal end joint portion 42 in the case of the gripping position P6 and the third intermediate joint portion 53 in the case of the gripping position P7) can be designed relatively shorter than in the guide wire 1S of the comparative example by providing the plurality of intermediate joint portions 50 in the guide wire 1. Therefore, in the guide wire 1, even when the above-described rotation following tests are carried out by setting the gripping positions between the first intermediate joint portion 51 and the second intermediate joint portion 52, and between the second intermediate joint portion 52 and the third intermediate joint portion 53, similarly good results are obtained.

### <Second Embodiment>

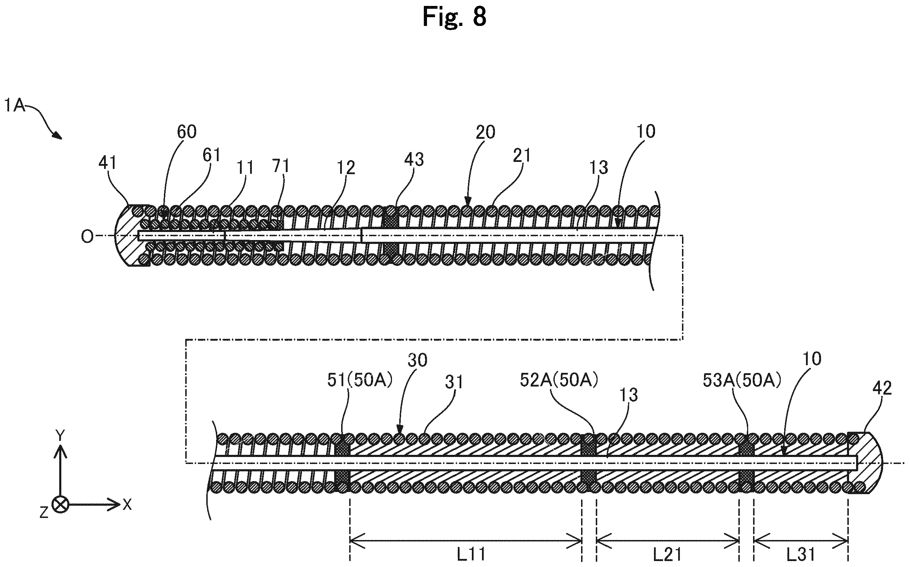

Fig. 8 is an explanatory diagram illustrating a configuration of a guide wire 1A according to a second embodiment. The guide wire 1A of the second embodiment includes a plurality of intermediate joint portions 50A, instead of the plurality of intermediate joint portions 50 described in the first embodiment. The plurality of intermediate joint portions 50A include first to third intermediate joint portions 51A to 53A having different arrangements in the longitudinal direction (the direction of the axial line O). Specifically, the length L11 between the proximal end of the first intermediate joint portion 51A and the distal end of the second intermediate joint portion 52A is longer than a length L21 between the proximal end of the second intermediate joint portion 52A and the distal end of the third intermediate joint portion 53A. Further, the length L21 is longer than a length L31 between the proximal end of the third intermediate joint portion 53A and the distal end EP of the proximal end joint portion 42 (L11 > L21 > L31). In other words, the remaining intermediate joint portions (the second intermediate joint portion 52A and the third intermediate joint portion 53A) are arranged at equal intervals between the first intermediate joint portion 51A arranged furthermost to the distal end side and the proximal end joint portion 42.

As described above, various modifications may be applied to the configuration of the plurality of intermediate joint portions 50A, and at least some of the intermediate joint portions of the plurality of intermediate joint portions 50A may not be arranged at equal intervals. In this case, the size relationship of the lengths L11, L21, and L31 described above can be freely changed. The guide wire 1A according to the second embodiment can also exhibit an effect similar to that of the first embodiment. Further, in the guide wire 1A of the second embodiment, the plurality of intermediate joint portions 50A can be easily manufactured, and the manufacturing cost of the guide wire 1A can be reduced.

### <Third Embodiment>

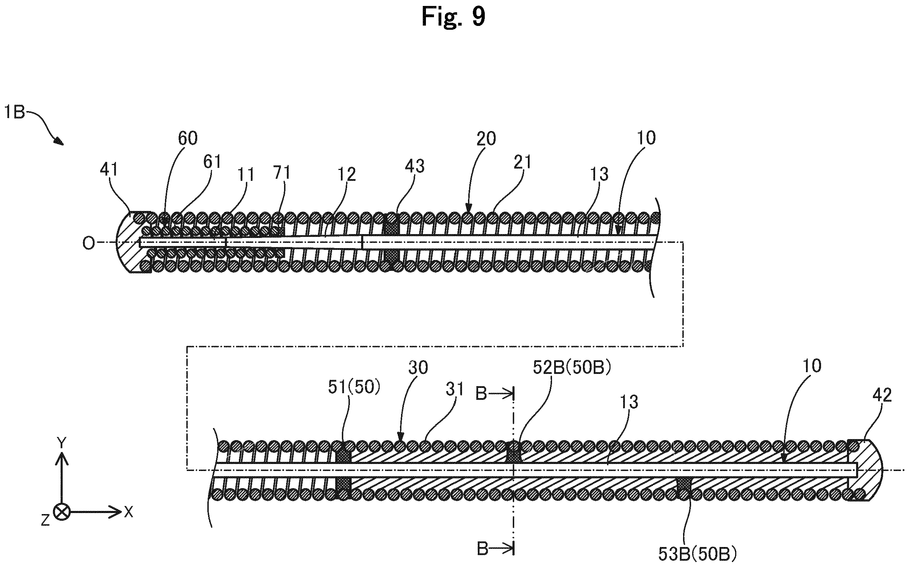

Fig. 9 is an explanatory diagram illustrating a configuration of a guide wire 1B according to a third embodiment. The guide wire 1B of the third embodiment includes a plurality of intermediate joint portions 50B instead of the plurality of intermediate joint portions 50 described in the first embodiment. The plurality of intermediate joint portions 50B include a second intermediate joint portion 52B instead of the second intermediate joint portion 52, and a third intermediate joint portion 53B instead of the third intermediate joint portion 53.

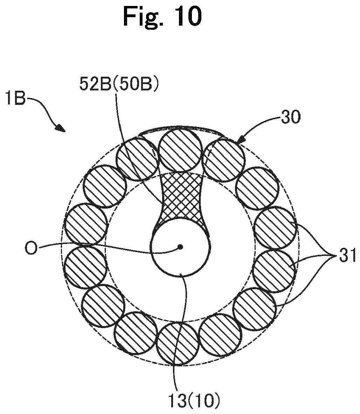

Fig. 10 is an explanatory diagram illustrating a cross-sectional configuration taken along line B-B of Fig. 9. As illustrated in Fig. 10, the second intermediate joint portion 52B is formed by welding a part of the large diameter portion 13 of the core shaft 10 in a circumferential direction and a part of the proximal end side coil body 30 in the circumferential direction. A range in which the core shaft 10 and the proximal end side coil body 30 are welded can be freely determined, and as illustrated in the drawing, the range may be about 30 degrees in the circumferential direction, or about 180 degrees, or about 360 degrees (that is, an aspect in which the core shaft 10 and the proximal end side coil body 30 are welded along the entire circumferential direction). Further, a welding length in a long axis direction (the direction of the axial line O) can also be freely determined.

Thus, various modifications may be applied to the configuration of the plurality of intermediate joint portions 50B, and at least some of the plurality of intermediate joint portions 50B may be formed by a means other than joining by a joining agent. As the means other than joining, a well-known means such as crimping can be utilized in addition to the above-described welding. Further, all of the plurality of intermediate joint portions 50B may be formed by a means other than joining, or at least some of the plurality of intermediate joint portions 50B may be formed by a means other than joining. The guide wire 1B according to the third embodiment can also exhibit an effect similar to that of the first embodiment. Further, in the guide wire 1B of the third embodiment, the plurality of intermediate joint portions 50B can be easily formed, and the manufacturing cost of the guide wire 1B can be reduced.

### <Fourth Embodiment>

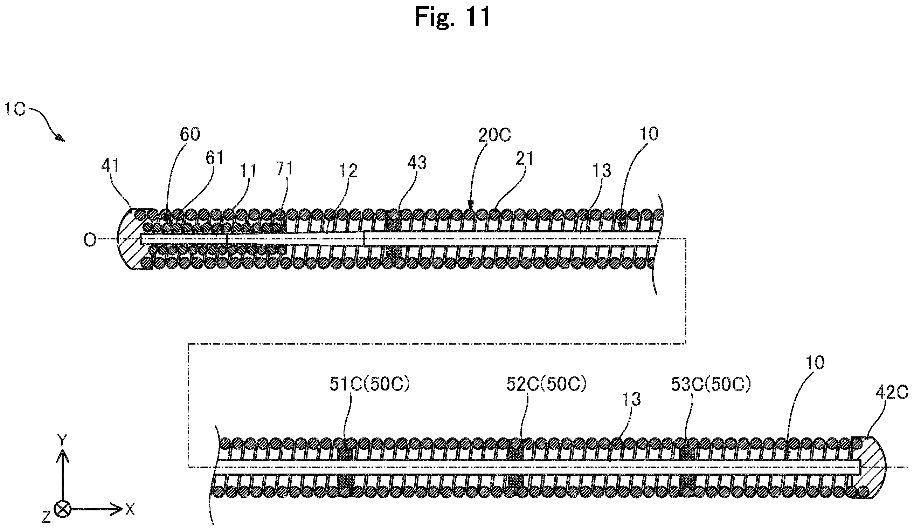

Fig. 11 is an explanatory diagram illustrating a configuration of a guide wire 1C according to a fourth embodiment. The guide wire 1C of the fourth embodiment does not include the proximal end side coil body 30 described in the first embodiment, includes a distal end side coil body 20C instead of the distal end side coil body 20, includes a plurality of intermediate joint portions 50C instead of the plurality of intermediate joint portions 50, and includes a proximal end joint portion 42C instead of the proximal end joint portion 42. The length of the distal end side coil body 20C in the long axis direction (the direction of the axial line O) is different from that of the first embodiment. Specifically, the distal end side coil body 20C is arranged so as to cover the entire guide wire 1 in the long axis direction. The plurality of intermediate joint portions 50C include first to third intermediate joint portions 51C to 53C. The first to third intermediate joint portions 51C to 53C respectively join the core shaft 10 and the distal end side coil body 20C. The proximal end joint portion 42C joins the proximal end portion of the core shaft 10 and the proximal end portion of the distal end side coil body 20C.

Thus, various modifications may be applied to the configuration of the guide wire 1C, and the coil body arranged on the outside may be configured from one type of coil. The coil body arranged on the outside may be a single-thread coil illustrated in Fig. 11, or may be a multi-thread coil in which a plurality of wires are wound into multiple threads. Further, the coil body arranged on the outside may be a single-thread twisted-wire coil formed by winding a twisted wire obtained by twisting a plurality of wires into a single thread, or may be a multi-thread twisted-wire coil formed by winding each twisted wire into multiple threads using a plurality of twisted wires obtained by twisting a plurality of wires. The guide wire 1C according to the fourth embodiment can also exhibit an effect similar to that of the first embodiment. Further, in the guide wire 1C of the fourth embodiment, the guide wire 1C can be easily formed, and the manufacturing cost of the guide wire 1C can be reduced.

### <Fifth Embodiment>

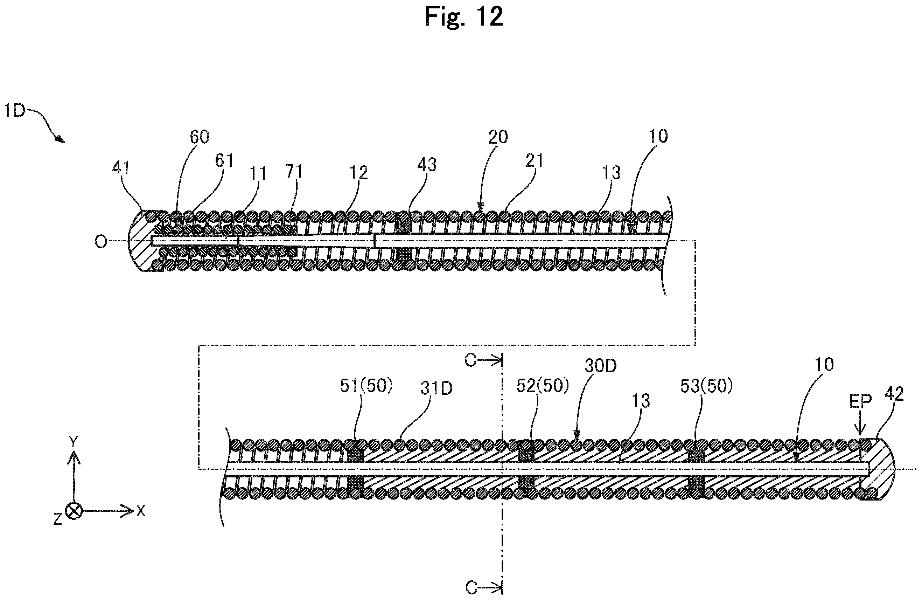

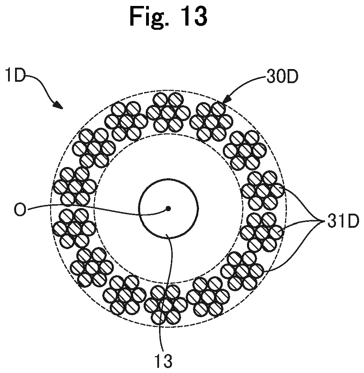

Fig. 12 is an explanatory diagram illustrating a configuration of a guide wire 1D according to a fifth embodiment. Fig. 13 is an explanatory diagram illustrating a cross-sectional configuration taken along line C-C of Fig. 12. The guide wire 1D of the fifth embodiment includes a proximal end side coil body 30D instead of the proximal end side coil body 30 described in the first embodiment. As illustrated in Fig. 13, the proximal end side coil body 30D is a multi-thread coil (multi-thread twisted-wire coil) in which a plurality of twisted wires 31D obtained by twisting a plurality of wires are wound. In the example of Fig. 13, the number of twisted wires 31D constituting the proximal end side coil body 30D is 14, and the number of wires constituting each of the twisted wires 31D is 7, but the number of twisted wires 31D and the number of wires constituting the twisted wires 31D can be freely determined. Further, the wires constituting the twisted wires 31D can be formed of a similar material as the wire 21. The material of the wires constituting the twisted wires 31D and the material of the wire 21 may be the same or different.

Thus, various modifications may be applied to the configuration of the proximal end side coil body 30D, and the proximal end side coil body 30D may be a multi-thread coil in which a plurality of the twisted wires 31D obtained by twisting a plurality of wires are wound. In the plurality of twisted wires 31D, the number of wires constituting some of the twisted wires 31D and the number of wires constituting the other ones of the twisted wires 31D may be different from each other. For example, some of the twisted wires 31D may be constituted by seven wires, and the other ones of the twisted wires 31D may be constituted by five wires. The guide wire 1D according to the fifth embodiment can also exhibit an effect similar to that of the first embodiment. Further, in the guide wire 1D of the fifth embodiment, the proximal end side coil body 30D is a multi-thread coil (multi-thread twisted-wire coil) in which the plurality of twisted wires 31D obtained by twisting a plurality of wires are wound. Thus, the torquability of the guide wire 1D can be further improved.

### <Sixth Embodiment>

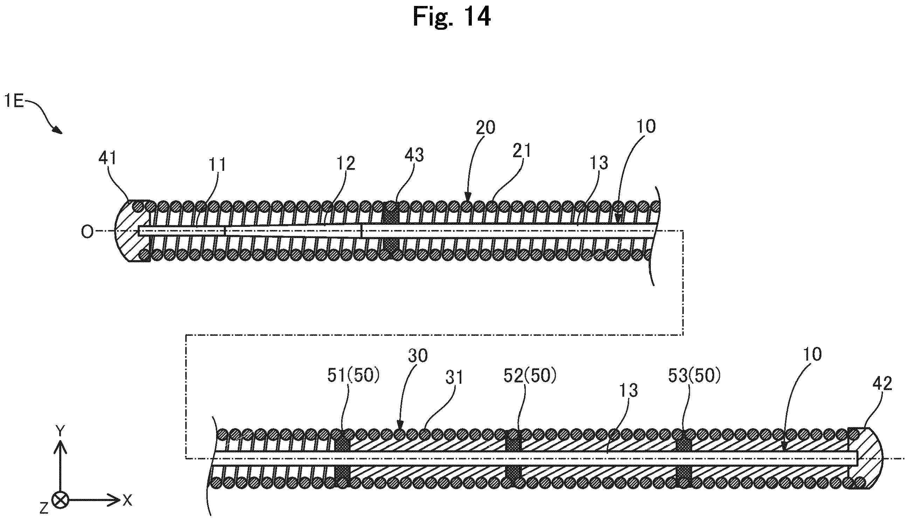

Fig. 14 is an explanatory diagram illustrating a configuration of a guide wire 1E according to a sixth embodiment. The guide wire 1E of the sixth embodiment does not include the inner coil body 60 described in the first embodiment. Thus, various modifications may be applied to the configuration of the guide wire 1E, and the guide wire 1E may not include at least a part of the configuration described in the first embodiment, and may include other configurations not described in the first embodiment. For example, the guide wire 1E may not include the first joint portion 43. The guide wire 1E according to the sixth embodiment can also exhibit an effect similar to that of the first embodiment.

### <Modifications of Embodiment>

The disclosed embodiments are not limited to the above-described embodiments, and may be implemented in various modes without departing from the spirit of the disclosed embodiments. The following modifications can be applied, for example.

### [First Modification]

In the above-described first to sixth embodiments, examples of the configurations of the guide wires 1 and 1A to 1E are described. However, the configuration of the guide wire 1 can be variously modified. For example, the guide wire 1 may further include a second core shaft (also referred to as a shaping ribbon) different from the core shaft 10. In this case, the proximal end portion of the second core shaft may be joined to the core shaft 10, and the distal end portion of the second core shaft may be fixed to the distal end side coil body 20 and the inner coil body 60 by the distal end joint portion 41. In a configuration in which the second core shaft is provided, it is possible to improve the shaping performance on the distal end side of the guide wire 1. For example, the guide wire may be designed as a product in which the distal end side is curved in advance.

For example, various modifications may be applied to the configuration of the core shaft 10, and the core shaft 10 may not include the small diameter portion 11, the reduced diameter portion 12, and the large diameter portion 13, may have a configuration in which the outer diameter of the entire longitudinal direction (the direction of the axial line O) is substantially the same, or may have a configuration in which the diameter of the entire longitudinal direction is reduced from the proximal end side to the distal end side. For example, the core shaft 10 may include a second reduced diameter portion in which the diameter is reduced from the proximal end side to the distal end side on the proximal end side of the large diameter portion 13, or may further include a second large diameter portion having a larger diameter than the large diameter portion 13 on the proximal end side of the second reduced diameter portion.

### [Second Modification]

In the first to sixth embodiments, an example of each configuration of the two coil bodies arranged on an outer side of the guide wires 1 and 1A to 1E (specifically, the distal end side coil bodies 20 and 20C, and the proximal end side coil bodies 30 and 30D) is described. However, various modifications may be applied to the configuration of the coil body. For example, the coil body may not cover the core shaft 10 in the entire longitudinal direction, and a part of the core shaft 10 on the proximal end side may not be covered by the coil body (Fig. 1: the proximal end side coil body 30) and may be exposed to the outside.

For example, the coil body arranged on an outer side of the guide wires 1 and 1A to 1E may be composed of one coil body or may be composed of three or more coil bodies. The coil body may be a single-thread coil formed by winding one wire into a single thread, or may be a multi-thread coil formed by winding a plurality of wires into multiple threads, or may be a single-thread twisted-wire coil formed by winding a twisted wire obtained by twisting a plurality of wires into a single thread, or may be a multi-thread twisted-wire coil (multi-thread coil) formed by winding each twisted wire into multiple threads using a plurality of twisted wires obtained by twisting a plurality of wires.

For example, the two coil bodies arranged on an outer side of the guide wires 1 and 1A to 1E (specifically, at least one of the distal end side coil bodies 20 and 20C, and at least one of the proximal end side coil bodies 30 and 30D) may have a dense winding configuration having no gap between adjacent wires, a sparse winding configuration having a gap between adjacent wires, or a combined configuration of the dense winding configuration and the sparse winding configuration. The coil body may include, for example, a resin layer coated with a hydrophobic resin material, a hydrophilic resin material, or a mixture thereof. For example, the transverse sectional shape of the wire of the coil body does not need to be substantially circular.

### [Third Modification]

In the first to sixth embodiments described above, examples of the configuration of the plurality of intermediate joint portions 50 and 50A to 50C are described. However, various modifications may be applied to the configuration of the plurality of intermediate joint portions 50. For example, the number of intermediate joint portions provided in the guide wires 1 and 1A to 1E is not limited to 3 as described above, and may be any number of 2 or more. For example, the shape of the intermediate joint portions can also be freely determined. For example, the interval between one intermediate joint portion and an adjacent intermediate joint portion may be 250 mm or more, and can be any value. For example, the interval between the one intermediate joint portion and the adjacent intermediate joint portion may not be based on an end face (the distal end/the proximal end) of the intermediate joint portion, but may be measured according to the position of an approximate central part of the intermediate joint portion in the longitudinal direction (the direction of the axial line O).

For example, the first intermediate joint portions 51 and 51A to 51C arranged furthermost to the distal end side among the plurality of intermediate joint portions 50 and 50A to 50C, may not join the proximal end portion of the distal end side coil body 20 and the distal end portion of the proximal end side coil body 30. In this case, the first intermediate joint portion 51 may be arranged on the distal end side of a boundary between the distal end side coil body 20 and the proximal end side coil body 30 (that is, the distal end side coil body 20), or may be arranged on the proximal end side of the boundary (that is, the proximal end side coil body 30).

### [Fourth Modification]

The configurations of the guide wires 1 and 1A to 1E of the first to sixth embodiments and the configurations of the guide wires 1 and 1A to 1E of the first to third modifications described above may be appropriately combined. For example, the guide wire 1C that does not include the proximal end side coil body 30 described in the fourth embodiment may be configured to include the plurality of intermediate joint portions 50 described in the second or third embodiment. For example, the guide wire 1D that includes the proximal end side coil body 30D described in the fifth embodiment may be configured to include the plurality of intermediate joint portions 50 described in the second or third embodiment. For example, the guide wire 1E that does not include the inner coil body 60 described in the sixth embodiment may be configured to include the plurality of intermediate joint portions 50 described in the second or third embodiment.

Although the aspects have been described based on the embodiments and the modifications, the embodiments of the above-described aspects are for facilitating understanding of the aspects, and do not limit the aspects. The aspects can be modified and improved without departing from the spirit of the aspects and the scope of the claims, and equivalent aspects are included in the aspects. Further, unless a technical feature is described as essential in the present specification, the technical feature may be omitted as appropriate.

### DESCRIPTION OF REFERENCE NUMERALS

1, 1A to 1E, 1b ... Guide wire
1S, 1Sa, 1Sb ... Guide wire of comparative example
10 ... Core shaft
11 ... Small diameter portion
12 ... Reduced diameter portion
13 ... Large diameter portion
20, 20C ... Distal end side coil body
21 ... Wire
30, 30D ... Proximal end side coil body
31 ... Wire
31D ... Twisted wire
41 ... Distal end joint portion
42, 42C ... Proximal end joint portion
43 ... First joint portion
50, 50A to 50C ... Intermediate joint portion
51, 51A to 51C ... First intermediate joint portion
52, 52A to 52C ... Second intermediate joint portion
53, 53A to 53C ... Third intermediate joint portion
60 ... Inner coil body
61 ... Wire
71 ... Second joint portion
81 ... Tube

## Claims

1. A guide wire comprising:
a core shaft;
a coil body wound around the entire core shaft;
a distal end joint portion that joins a distal end portion of the core shaft and a distal end portion of the coil body;
a proximal end joint portion that joins a proximal end portion of the core shaft and a proximal end portion of the coil body; and
a plurality of intermediate joint portions that are each arranged on a distal end side of a distal end of the proximal end joint portion and each join the core shaft and the coil body.

2. The guide wire according to claim 1, wherein
between the proximal end joint portion and the intermediate joint portion arranged furthermost to the distal end side among the plurality of intermediate joint portions, remaining ones of the intermediate joint portions are arranged at equal intervals.

3. The guide wire according to claim 1 or claim 2, wherein
the interval between one of the intermediate joint portions and an adjacent one of the intermediate joint portions is 250 mm or less.

4. The guide wire according to any one of claims 1 to 3, wherein
at least a part of the core shaft in a circumferential direction and at least a part of the coil body in the circumferential direction are welded in the intermediate joint portion.

5. The guide wire according to any one of claims 1 to 4, wherein the coil body includes:
a distal end side coil body arranged on the distal end side; and
a proximal end side coil body arranged on a proximal end side, and
the intermediate joint portion arranged furthermost to the distal end side among the plurality of intermediate joint portions joins a proximal end portion of the distal end side coil body and a distal end portion of the proximal end side coil body.

6. The guide wire according to claim 5, wherein
the proximal end side coil body is a multi-thread coil in which a plurality of wires are wound into multiple threads.

7. The guide wire according to claim 5, wherein
the proximal end side coil body is a multi-thread coil in which a plurality of twisted wires obtained by twisting a plurality of wires are wound.

8. The guide wire according to any one of claims 5 to 7, wherein
the distal end side coil body is a single-thread coil in which one wire is wound into a single thread.
